(19) Europäisches Patentamt
European Patent Office
Office européen des brevets

(11) **EP 4 141 584 A1**

(12) **EUROPEAN PATENT APPLICATION**
published in accordance with Art. 153(4) EPC

(43) Date of publication:
**01.03.2023 Bulletin 2023/09**

(21) Application number: **21792125.3**

(22) Date of filing: **31.03.2021**

(51) International Patent Classification (IPC):
***G05B 17/02*** (2006.01)

(52) Cooperative Patent Classification (CPC):
**G05B 17/02**

(86) International application number:
**PCT/CN2021/084557**

(87) International publication number:
**WO 2021/213166 (28.10.2021 Gazette 2021/43)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **22.04.2020 CN 202010322898
22.04.2020 CN 202010322919**

(71) Applicant: **The Fourth Paradigm (Beijing) Tech Co
Ltd
Beijing 100085 (CN)**

(72) Inventors:
• **WANG, Mengshuo
  Beijing 100085 (CN)**
• **TU, Weiwei
  Beijing 100085 (CN)**

(74) Representative: **Müller Hoffmann & Partner
Patentanwälte mbB
St.-Martin-Strasse 58
81541 München (DE)**

(54) **SIMULATION SYSTEM AND SIMULATION METHOD, AND EPIDEMIC DEDUCTION SIMULATION SYSTEM AND SIMULATION METHOD**

(57)     Disclosed are a simulation system and a simulation method, and an epidemic deduction simulation system and simulation method. The simulation system comprises: a user interface module(101), which is configured to receive an external input(501); a control module(102), which is configured to control, on the basis of the external input, a behavior described in a preset simulation logic program(502), wherein in the preset simulation logic program, a state in a simulated item and a behavior which drives the state to change are described in the form of a code, and when the external input includes a learnable parameter, the control module acquires an optimization value of the learnable parameter from an optimization module, and executes, by means of taking the optimization value as a value of the learnable parameter, a step of controlling the behavior described in the preset simulation logic program on the basis of the external input; a simulation module(103), which is configured to run the preset simulation logic program in response to the control of the control module(503); and the optimization module(104), which is configured to perform optimization learning on the value of the learnable parameter on the basis of simulation result data output by the simulation module(504).

FIG. 5

## Description

This application claims priority to Chinese patent application No. 202010322898.X, entitled "SIMULATION SYSTEM AND SIMULATION METHOD" filed on April 22, 2020 and Chinese patent application No. 202010322919.8, entitled "EPIDEMIC DEDUCTION SIMULATION SYSTEM AND SIMULATION METHOD" filed on April 22, 2020, the disclosure of the above applications is incorporated by reference into the present application.

## TECHNICAL FIELD

The present disclosure generally relates to the field of artificial intelligence, and more particularly, to a data-driven simulation system and simulation method, and an epidemic deduction simulation system and simulation method.

## BACKGROUND

Simulation (or emulation) is an approximation or imitation of how a process or a system works. A simulator is a kind of software that has an ability to simulate an operation of a process or a system over time. The simulator needs to extract key states involved in the operation of the system, and use a program to simulate changes of these states (which can be called behaviors of the system). The simulator can be used for system simulation, analysis, tuning, testing, teaching, etc., and has a wide range of applications.

A traditional simulator abstracts system behaviors into mathematical formulas or solutions of mathematical problems through domain knowledge, while determines parameters involved in the mathematical formulas or problems, and further converts them into computer-understandable codes, thereby realizing a simulation function. Here, the mathematical formulas (problems) and their corresponding codes may be simply referred to as rules, and the rules may involve corresponding parameters. For example, in a vehicle simulator, a speed state of the vehicle can be updated by simulating an acceleration process of the vehicle using an acceleration formula; in an electromagnetic simulator, system states such as electric field intensity and magnetic field intensity can be obtained by solving Maxwell's equations. Here, the key states are the speed of the vehicle and the intensity of the electromagnetic field, respectively. The system behaviors are abstracted into the acceleration formula or solving Maxwell's equations. The involved parameters are an acceleration of the vehicle or a dielectric constant of a medium.

However, it is difficult to build the traditional simulator in many application scenarios. On the one hand, the traditional simulator relies on rules and parameters provided by domain knowledge, but in actual situations, the following situations may be encountered: 1. Domain knowledge about rules is lacking. For example, in a supply chain scenario, it is necessary to simulate an order arrival behavior, but the order arrival is a result of the mutual influence of various factors, and the process is too complicated to be abstracted into simple rules. 2. Domain knowledge about parameters is lacking. For example, in an epidemic deduction scenario, a key parameter of an infection number (how many susceptible people can be infected by one infected person) needs to be determined, but the estimation of the infection number needs to wait for a detailed long-term study of the infectious disease, and the estimation may be inaccurate. Both of the above cases (lack of rules, lack of parameters) can make it impossible to build the traditional simulator. This largely limits the applicability of the traditional simulator. On the other hand, the simulator may simulate a target system at a certain level of abstraction. For example, the vehicle simulator can simulate the acceleration process of the vehicle from a higher level (such as using acceleration parameters and applying the acceleration formula), or it can simulate from a lower level (such as considering a series of complex underlying mechanisms such as fuel combustion, pushing pistons, force transmission, etc.). Therefore, under the premise of a given abstraction level, the traditional simulator cannot utilize useful data available in reality, and the accuracy of the traditional simulator (the ability to reflect a real system) is limited by accuracy and completeness of domain knowledge.

## SUMMARY

Exemplary embodiments of the present disclosure aim to overcome the above drawbacks of the traditional simulator that is limited due to lack of rules or parameters and limited in precision.

According to a first aspect of the present disclosure, a system including at least one computing device and at least one storing device is provided, wherein the at least one storing device stores instructions that, when executed by the at least one computing device, cause the at least one computing device to perform a simulation method, the simulation method includes: receiving an external input; controlling a behavior described in a preset simulation logic program based on the external input, wherein the preset simulation logic program describes a state in a simulated item and a behavior that drives a change in the state in code, wherein when the external input includes a learnable parameter, an optimized value of the learnable parameter is obtained, and the step of controlling the behavior described in the preset simulation logic program based on the external input is performed by using the optimized value as a value of the learnable parameter; in response to the controlling, running the preset simulation logic program; optimizing the value of the learnable parameter based on output simulation result data.

According to a second aspect of the present disclosure, a simulation method performed by a system including at least one computing device and at least one storing device is provided, the at least one storing device stores instructions that, when executed by the at least one computing device, cause the at least one computing device to perform the simulation method, the simulation method includes: receiving an external input; controlling a behavior described in a preset simulation logic program based on the external input, wherein the preset simulation logic program describes a state in a simulated item and a behavior that drives a change in the state in code, wherein when the external input includes a learnable parameter, an optimized value of the learnable parameter is obtained, and the step of controlling the behavior described in the preset simulation logic program based on the external input is performed by using the optimized value as a value of the learnable parameter; in response to the controlling, running the preset simulation logic program; optimizing the value of the learnable parameter based on output simulation result data.

According to a third aspect of the present disclosure, a simulation system is provided, the simulation system includes: a user interface module configured to receive an external input; a control module configured to control a behavior described in a preset simulation logic program based on the external input, wherein the preset simulation logic program describes a state in a simulated item and a behavior that drives a change in the state in code, wherein when the external input includes a learnable parameter, an optimized value of the learnable parameter is obtained, and the step of controlling the behavior described in the preset simulation logic program based on the external input is performed by using the optimized value as a value of the learnable parameter; a simulation module configured to run the preset simulation logic program in response to the controlling; an optimization module configured to optimize the value of the learnable parameter based on output simulation result data.

According to a fourth aspect of the present disclosure, a system including at least one computing device and at least one storing device is provided, wherein the at least one storing device stores instructions that, when executed by the at least one computing device, cause the at least one computing device to perform a simulation method, the simulation method includes: receiving an external input; controlling a behavior described in a preset simulation logic program based on the external input, wherein the preset simulation logic program describes a state in epidemic development and a behavior that drives a change in the state in the epidemic development in code, wherein when the external input includes a learnable parameter, an optimized value of the learnable parameter is obtained, and the step of controlling the behavior described in the preset simulation logic program based on the external input is performed by using the optimized value as a value of the learnable parameter; in response to the controlling, running the preset simulation logic program; optimizing the value of the learnable parameter based on output simulation result data.

According to a fifth aspect of the present disclosure, a simulation method performed by a system including at least one computing device and at least one storing device is provided, the at least one storing device stores instructions that, when executed by the at least one computing device, cause the at least one computing device to perform the simulation method, the simulation method includes: receiving an external input; controlling a behavior described in a preset simulation logic program based on the external input, wherein the preset simulation logic program describes a state in epidemic development and a behavior that drives a change in the state in the epidemic development in code, wherein when the external input includes a learnable parameter, an optimized value of the learnable parameter is obtained, and the step of controlling the behavior described in the preset simulation logic program based on the external input is performed by using the optimized value as a value of the learnable parameter; in response to the controlling, running the preset simulation logic program; optimizing the value of the learnable parameter based on output simulation result data.

According to a sixth aspect of the present disclosure, a epidemic deduction simulation system is provided, the epidemic deduction simulation system includes: a user interface module configured to receive an external input; a control module configured to control a behavior described in a preset simulation logic program based on the external input, wherein the preset simulation logic program describes a state in epidemic development and a behavior that drives a change in the state in the epidemic development in code, wherein when the external input includes a learnable parameter, an optimized value of the learnable parameter is obtained, and the step of controlling the behavior described in the preset simulation logic program based on the external input is performed by using the optimized value as a value of the learnable parameter; a simulation module configured to run the preset simulation logic program in response to the controlling; an optimization module configured to optimize the value of the learnable parameter based on output simulation result data.

According to a seventh aspect of the present disclosure, a computer readable storage medium storing instructions that, when executed by at least one computing device, cause the at least one computing device to perform the simulation method according to the present disclosure.

According to the simulation system and simulation method of the present disclosure, real data (for example, historical data) can be utilized to determine parameters required for simulation that cannot be obtained from domain knowledge temporarily by fitting the real data, thereby reducing the dependence of the simulation system on domain knowledge, expanding the applicable field and scope of the simulation system, meeting the simulation requirements of many different scenarios, and improving the simulation accuracy.

In addition, according to the epidemic deduction simulation system and simulation method suitable for epidemic deduction scenarios of the present disclosure, compared with traditional epidemic models (e.g., SIR, SEIR), a more complex epidemic deduction simulation logic can be realized, and real epidemic data can be utilized to determine epidemic deduction parameters required for simulation that cannot be obtained from epidemic domain knowledge temporarily by fitting the real epidemic data, so as to obtain more accurate epidemic deduction simulation results.

**BRIEF DESCRIPTION**

These and/or other aspects and advantages of the present disclosure will become more apparent and more readily understood from the following detailed description of embodiments of the present disclosure, taken in conjunction with the accompanying drawings, wherein:

FIG. 1 is a block diagram illustrating a simulation system according to an exemplary embodiment of the present disclosure;
FIG. 2 is a logical schematic diagram illustrating a simulation system according to an exemplary embodiment of the present disclosure;
FIG. 3 is a schematic diagram illustrating a relationship between a control module and a simulation logic program according to an exemplary embodiment of the present disclosure;
FIG. 4 shows an architectural diagram of a simulation system according to an exemplary embodiment of the present disclosure;
FIG. 5 is a flowchart illustrating a simulation method according to an exemplary embodiment of the present disclosure;
FIG. 6 is a schematic diagram illustrating an epidemic deduction simulation logic according to an exemplary embodiment of the present disclosure.

**DETAILED DESCRIPTION**

In order for those skilled in the art to better understand the present disclosure, the exemplary embodiments of the present disclosure will be described in further detail below with reference to the accompanying drawings and specific embodiments.

It should be noted here that "at least one of several items" in the present disclosure all means including three parallel situations of "any one of the several items", "a combination of any of the several items" and "the whole of the several items". In the present disclosure, "and/or" all means at least one of two or more items connected by it. For example, "including at least one of A and B" and "including A and/or B" include the following three parallel situations: (1) including A; (2) including B; (3) including A and B. For another example, "performing at least one of step 1 and step 2", "performing step 1 and/or step 2" means the following three parallel situations: (1) performing step 1; (2) performing step 2; (3) performing step 1 and step 2. That is, "A and/or B" can also be expressed as "at least one of A and B", and "performing step 1 and/or step 2" can also be expressed as "performing at least one of step 1 and step 2".

The basic idea of the present disclosure is to use a data-driven approach to reduce the dependence of the simulation system on domain knowledge, thereby expanding the scope of application of the simulation system and improving the accuracy of the simulation system. According to this basic idea, a general simulation system framework is proposed and may be applied to epidemic deduction scenarios. The simulation system framework can include three basic parts, namely, a simulation module for running a data-driven based simulation logic, a control module for flexibly intervening the simulation logic, and an optimization module for learning some parameters required for simulation in a data-driven way. Therefore, when designing the simulation system framework, the three parts may be designed independently on the premise of clarifying the overall design (such as sorting out available domain knowledge and data, determining abstraction levels of the simulation system, etc.). Such a simulation system framework is developed using the processes of start development, overall design, three-part independent design development, testing and iteration, and development completion.

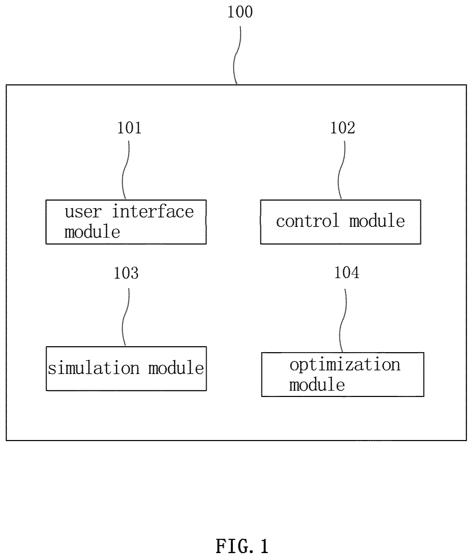

The simulation system and the simulation method according to exemplary embodiments of the present disclosure will be described in detail below with reference to FIGS. 1 to 6.

FIG. 1 is a block diagram illustrating a simulation system according to an exemplary embodiment of the present disclosure.

Referring to FIG. 1, a simulation system 100 according to an exemplary embodiment of the present disclosure may include a user interface module 101, a control module 102, a simulation module 103 and an optimization module 104.

According to an exemplary embodiment of the present disclosure, the user interface module 101 is a module for receiving data input from the outside. In addition, the user interface module 101 may also receive any other external user input, control input, and the like.

The simulation module 103 is a module for storing and running a preset simulation logic program. Here, the preset simulation logic program describes a state in a simulated item and a behavior that drives a change in the state in code. The state in the simulated item may include one or more states, and the behavior that drives the change in the state may also include one or more behaviors, the behavior involves at least one of a rule, a parameter and data. For example, in the case where the simulation system 100 is a vehicle speed simulation system, the preset simulation logic program describes a state in a change of a vehicle speed (e.g., a speed of a vehicle) and a behavior that drives a change in the state in the change of the vehicle speed in code (e.g., an acceleration of the vehicle). For another example, in the case where the simulation system 100 is an epidemic deduction simulation system, the preset simulation logic program describes a state in epidemic development (for example, a number of confirmed patients, etc.) and a behavior that drives a change in the state in the epidemic development in code (e.g., a confirmed behavior, etc.). These two application scenarios will be described in detail below.

The control module 102 is a module for finely controlling the simulation logic program by a user or developer. For example, the control module 102 may convert an external input received through the user interface module 101 into rules, parameters and data required by the simulation logic program according to its own logic based on the external input, or the control module 102 may directly intervene (e.g., modify) the state or the behavior in the simulation logic program. In addition, the control module 102 may also perform a corresponding control according to other users' input or control input.

The optimization module 104 is a module for optimizing learnable parameters required by the simulation system 100 to improve the simulation accuracy of the simulation system 100. The process of optimizing the parameter is a process of learning the parameter, through which the optimized parameter can be obtained.

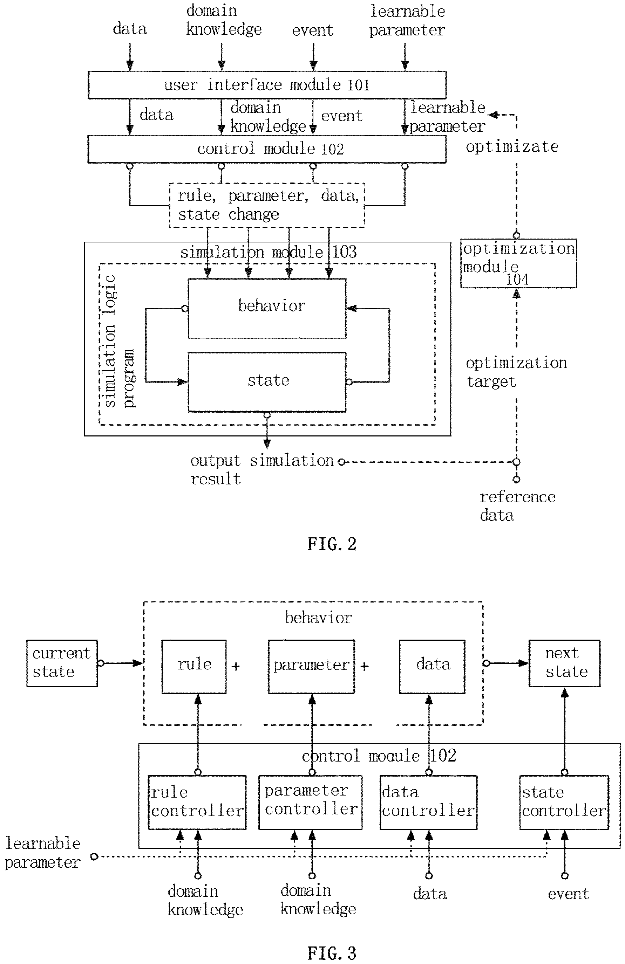

Specific operations of the simulation system 100 according to an exemplary embodiment of the present disclosure will be described in detail below with reference to FIGS. 2 and 3.

FIG. 2 is a logical schematic diagram illustrating a simulation system 100 according to an exemplary embodiment of the present disclosure.

Referring to FIG. 2, the user interface module 101 may receive an external input. Here, the external input may include at least one of domain knowledge, data and an event related to a simulated item. In addition, the external input may also include a learnable parameter. Here, the learnable parameter refers to at least one parameter related to the simulated item that cannot be directly obtained from the domain knowledge, the data, or the event.

The control module 102 may control the behavior described in the preset simulation logic program based on the external input received through the user interface module 101.

The simulation module 103 may run the preset simulation logic program in response to the control of the control module 102.

The optimization module 104 may optimize the value of the learnable parameter based on the simulation result data output by the simulation module 103.

According to an exemplary embodiment of the present disclosure, the control module 102 converts the external input (e.g., at least one of the domain knowledge, the data, the event and the learnable parameter) into at least one of the rule, the parameter and the data involved in the behavior described in the preset simulation logic program and sends the converted at least one of the rule, the parameter and the data to the simulation module 103. The simulation module 103 can apply the converted at least one of the rule, the parameter and the data to the preset simulation logic program and running the preset simulation logic program, to obtain and output the simulation result data.

According to an exemplary embodiment of the present disclosure, the control module 102 may also directly intervene the behavior described in the simulation logic program. For example, the user interface module 101 may also receive a user input for modifying the behavior in the simulation logic program. The control module 102 may modify at least one of the rule, the parameter and the data according to the user input and send the modified at least one of the rule, the parameter and the data to the simulation module 103. The simulation module 103 may apply the modified at least one of the rule, the parameter and the data to the preset simulation logic program and running the preset simulation logic program, to obtain and output the simulation result data.

According to an exemplary embodiment of the present disclosure, the control module 102 may also directly intervene a change of the state described in the simulation logic program. For example, the user interface module 101 may also receive a user input for modifying the state described in the simulation logic program. The control module 102 may modify a change in at least one of the states described in the simulation logic program based on the user input.

For example, referring to FIG. 3, FIG. 3 is a schematic diagram illustrating a relationship between the control module 102 and the simulation logic program according to an exemplary embodiment of the present disclosure. The control module 102 may include one or more controllers, for example, may include at least one of a rule controller, a parameter controller, a data controller, and a state controller. The rule controller may convert at least one of domain knowledge and a learnable parameter in an external input into a rule involved in the behavior in the simulation logic program, or may intervene (e.g., modify) the rule involved in the behavior in the simulation logic program based on a user input. The parameter controller can convert at least one of domain knowledge and a learnable parameter in an external input into a parameter involved in the behavior in the simulation logic program, or may intervene (e.g., modify) the parameter involved in the behavior in the simulation logic program based on a user input. The data controller can convert at least one of data and a learnable parameter in an external input into a parameter involved in the behavior in the simulated logic program, or can intervene (e.g., modify) data involved in the behavior in the simulation logic program based on a user input. The simulation module 103 can run the simulation logic program in response to the control of the rule controller, the parameter controller and the data controller. During the process of running the simulation logic program, the rule, the parameter and the data involved in the behavior of the simulation logic program can drive the state in the simulation logic program to change from a current state to a next state based on the control of the rule controller, the parameter controller and the data controller. In addition, the state controller may directly intervene the change in the state in the simulation logic program (i.e., directly modify the converted state) based on at least one of an event and a learnable parameter in a user input. Of course, the above relationship is an exemplary, and the present application does not limit the input, internal structure and conversion logic of the control module 102.

Referring back to FIG. 2, the user interface module 101 may also receive a user input for initializing the simulation system 100 according to an exemplary embodiment of the present disclosure. The control module 102 may set an initial value of the state in the simulation logic program according to the user input. In addition, when the external input includes the learnable parameter, the control module 102 may set the value of the learnable parameter according to the user input. In addition, when the initial value of the state in the simulation logic program cannot be directly obtained from the domain knowledge, the data or the event, the initial value of the state in the simulation logic program may be used as a learnable parameter.

After completing the initialization, when the control module 102 controls the behavior in the simulation logic program based on the external input (e.g., at least one of domain knowledge, data, events, and learnable parameters), the simulation module 103 can run the simulation logic program, such that the behavior in the simulation logic program drives the state in the simulation logic program to change from an initial state to a next state. Therefore, the simulation module 103 may output the changed state as simulation result data. When the external input includes the learnable parameter, the optimization module 104 may optimize the value of the learnable parameter based on the simulation result data output by the simulation module 103. When performing a next simulation, the user interface module 101 may re-receive an external input (e.g., re-receive at least one of domain knowledge, data, and events), and the control module 102 may obtain the re-received external input from the user interface module 101, obtain the optimized value of the learnable parameter from the optimization module 104 and control the behavior in the simulation logic program based on the re-received external input by using the optimized value as the value of the learnable parameter in the external input. Then, the simulation module 103 can run the simulation logic program again and output the simulation result data, based on this control of the control module 102, and the optimization module 104 can optimize the current value of the learnable parameter based on the simulation result data again. So on and so forth.

That is, in the case where the external input includes the learnable parameter, an operation mode of the simulation system 100 may include an optimization mode and a simulation mode. In the optimization mode, the user interface module 101, the control module 102, the simulation module 103, and the optimization module 104 may perform operations iteratively to obtain a final optimized value of the learnable parameter. In the simulation mode, the control module 102 performs the step of controlling the behavior described in the preset simulation logic program based on the external input by taking the final optimized value of the learnable parameter as the value of the learnable parameter, and in response to the control of the control module 102, the simulation module 103 may run the preset simulation logic program, and output accurate simulation result data.

It may be seen that even if the simulation system 100 needs some parameters (i.e., learnable parameters) that cannot be obtained directly or through simple operations from domain knowledge or data, the values of these parameters may be manually set first, and then the optimized values of these parameters may be obtained through simulation and optimization of the simulation system 100, so that the simulation result of the simulation system 100 tend to be accurate. In other words, the data-driven method is used to reduce the dependence of the simulation system 100 on domain knowledge or data and improve the accuracy of the simulation system 100.

According to an exemplary embodiment of the present disclosure, the user interface module 101 can receive an external input in real time, the control module 102 controls the behavior described in the preset simulation logic program based on the real-time external input, and in response to the control of the control module 102, the simulation module 103 runs the preset simulation logic program in real time, and the optimization module 104 optimizes the value of the learnable parameter based on the simulation result data output by the simulation module 103 in real time. For example, the user interface module 101, the control module 102, the simulation module 103, and the optimization module 104 may perform operations iteratively at a predetermined time interval. For another example, the user interface module 101, the control module 102 and the simulation module 103 may perform operations at a first predetermined time interval, and the optimization module 104 may perform operations at a second predetermined time interval, wherein the second predetermined time interval is longer than the first predetermined time interval. For example, in the application scenario of epidemic deduction, the user interface module 101, the control module 102 and the simulation module 103 can perform operations every day, and the epidemic simulation result data is output every day, while the optimization module 104 can perform operations every ten days, that is, the optimization module 104 can optimize the learnable parameter based on the ten-day epidemic simulation result data, which will be described in detail later.

According to an exemplary embodiment of the present disclosure, the user interface module 101 may also receive reference data of the simulated item, such as historical data or real data of the simulated item. The optimization module 104 can calculate an optimization target based on the simulation result data output by the simulation module 103 and the reference data of the simulated item, and optimize the optimization target through an optimization algorithm, thereby tuning the value of the learnable parameter.

Here, the learnable parameter may include a type and a value range, where the type is used to describe the type of the learnable parameter, for example, an integer (1, 2, 3), a floating-point number (3 .1415926 ), a string (such as "+" "-"), the value range is a range of all possible values of the learnable parameter corresponding to the type (for example , the type of the learnable parameter is a floating-point number, and the value range is [ 0, 100] ).

The optimization target is a value that may be quantitatively calculated and influenced by the learnable parameter. The calculation of the optimization target may involve, but is not limited to simulation result, data, and domain knowledge. This application does not limit the calculation method.

The optimization algorithm is an algorithm that maximizes/minimizes the optimization target as needed by tuning the learnable parameter ("decision variable" for optimization algorithms). Different optimizers can adopt different optimization algorithms to solve different problems (determined by learnable parameters and optimization targets). Optimization algorithms include, but not limited to, gradient descent algorithms, Bayesian optimization algorithms, evolutionary algorithms, and machine learning algorithms. This application does not limit the optimization algorithm. In addition, in the case of a machine learning algorithm, the learnable parameter may be a parameter involved in a machine learning model, the optimization target may be a loss function related to the machine learning model, and the optimization algorithm may be a training algorithm corresponding to the machine learning model.

For example, the optimization module 104 compares the simulation result data output by the simulation module 103 with the reference data of the simulated item, and based on the comparison result as the optimization target, optimizes the learnable parameter through an optimization algorithm.

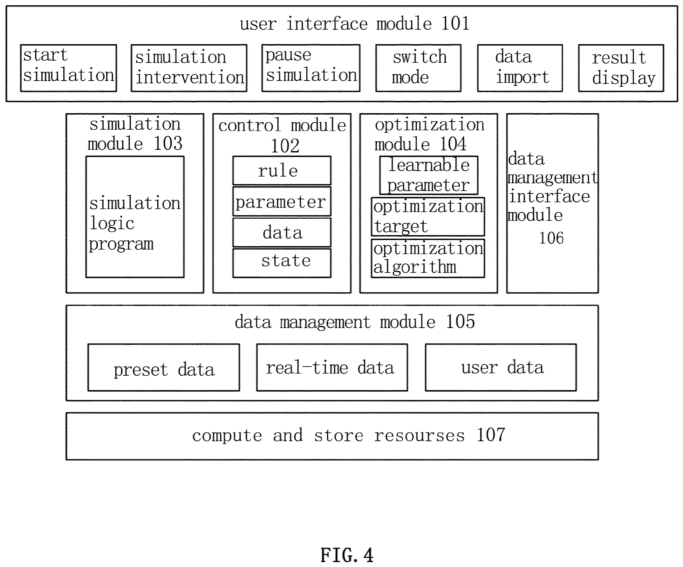

In addition, according to an exemplary embodiment of the present disclosure, the simulation system 100 may include a data management module 105 and a data management interface module 106 in addition to the user interface module 101, the control module 102, the simulation module 103 and the optimization module 104. In addition, the simulation system 100 may also include a computation and storage engine module 107. As shown in FIG. 4, FIG. 4 shows an architectural diagram of a simulation system 100 according to an exemplary embodiment of the present disclosure.

Specifically, referring to FIG. 4, the user interface module 101 may receive a control input. For example, the control input includes at least one of a start simulation input for starting a simulation, a simulation intervention input for intervening a simulation, a pause (or end) simulation input for pausing (or ending) a simulation, a data import input for importing data, a switch mode input for switching an optimization mode and a simulation mode and a result display input for displaying a result, but not limited to this.

According to an exemplary embodiment of the present disclosure, the user interface module 101 may transmit the received control input to the control module 102, and the control module 102 performs a corresponding control according to the received control input.

According to an exemplary embodiment of the present disclosure, the user interface module 101 may directly perform a corresponding control according to the received control input. In addition, the user interface module 101 can also be used as a display module for displaying a user interface. The user interface module 101 may also be implemented by a touch-sensitive screen. The user interface displayed by the user interface module 101 may include a button for receiving the control input, data received by the user interface module 101, and simulation-related data. Here, the data received by the user interface module 101 may include any input data such as the external input, the user input for modifying the behavior or state, the initial value of the state and the value of the learnable parameter as described above, and the like. The simulation-related data may include at least one of a current mode (e.g., an optimization mode or a simulation mode), simulation result data, and reference data of the simulated item.

The data management module 105 may store and manage data related to the simulation system 100. For example, these data may include preset data, real-time data, and user data. Here, the preset data may refer to data used by the developer when developing the simulation system 100, which may be provided to the customer together with the simulation system 100. The real-time data may refer to data that changes over time and is continuously updated during the use of the simulation system 100. The user data may refer to data provided by a user. These data may be received through the user interface module 101, or may be pre-stored, or may be transmitted and received through a network, etc., which is not limited in this application. The data management interface module 106 can import at least one piece of data in the data management module 105 into the control module 102. For example, the data management module 105 may store and manage external inputs received through the user interface module 101 and for example, in response to a control input through the user interface module 101, the data management interface module 106 imports the external input to the control module 102.

The computation and storage engine module 107 may support computing and storage requirements of the simulation system 100. The computation and storage engine module 107 may provide the simulation system 100 with underlying resources, including computing resources and storage resources. The computing resources may include algorithm libraries, computing frameworks, computing hardware, and the like. The storage resources may include database software, memory hardware, distributed storage servers, and the Internet and so on.

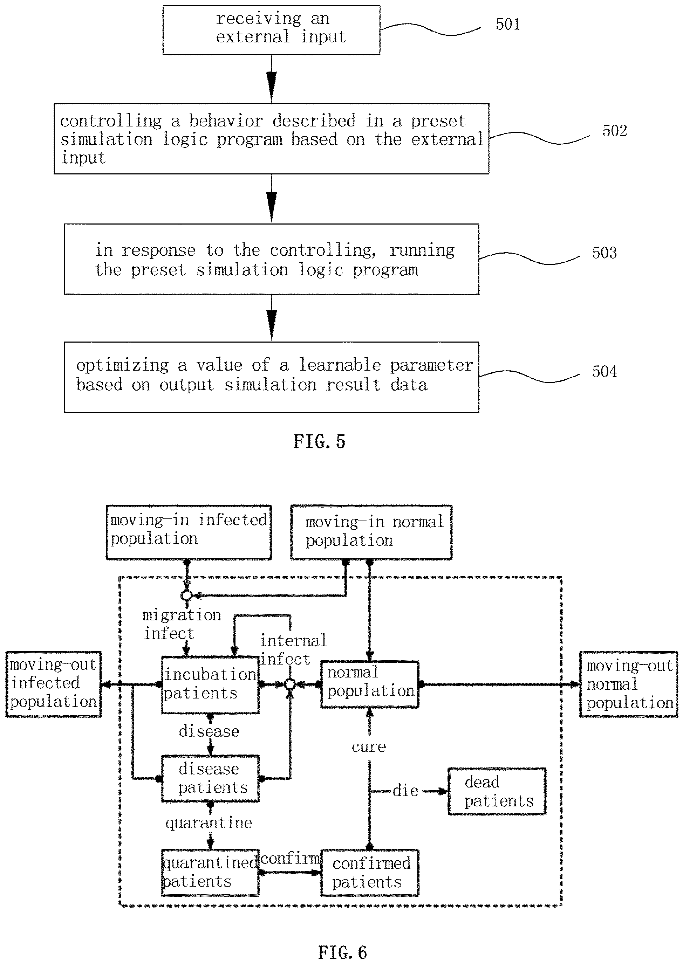

FIG. 5 is a flowchart illustrating a simulation method according to an exemplary embodiment of the present disclosure. The simulation method according to the exemplary embodiment of the present disclosure may be implemented by the simulation system 100 according to the exemplary embodiment of the present disclosure as described above, and may also be implemented by a system including at least one computing device and at least one storage device storing instructions.

As shown in FIG. 5, in step 501, an external input may be received. Here, the external input may include at least one of domain knowledge, data, and an event related to a simulated item. In addition, the external input may also include a learnable parameter. Here, the learnable parameter refers to at least one parameter related to the simulated item that cannot be directly obtained from the domain knowledge, the data, or the event.

In step 502, a behavior described in a preset simulation logic program may be controlled based on the external input, wherein the preset simulation logic program describes a state in a simulated item and a behavior that drives a change in the state in code. Here, the behavior may involve at least one of a rule, a parameter and data. For example, in the case of a vehicle speed simulation, the preset simulation logic program describes a state in a change of a vehicle speed (e.g., a speed of a vehicle) and a behavior that drives a change in the state in the change of the vehicle speed in code (e.g., an acceleration of the vehicle). For another example, in the case of an epidemic deduction simulation, the preset simulation logic program describes a state in epidemic development (for example, a number of confirmed patients, etc.) and a behavior that drives a change in the state in the epidemic development in code (e.g., a confirmed behavior, etc.). These two application scenarios will be described in detail below.

In step 503, the preset simulation logic program may be run in response to the controlling.

In step 504, a value of the learnable parameter may be optimized based on output simulation result data.

According to an exemplary embodiment of the present disclosure, in step 502, the external input (e.g., at least one of the domain knowledge, the data, the event and the learnable parameter) may be converted into at least one of the rule, the parameter and the data involved in the behavior in the preset simulation logic program. Subsequently, in step 503, the converted at least one of the rule, the parameter and the data may be applied to the preset simulation logic program and the preset simulation logic program is run to obtain and output the simulation result data.

Furthermore, according to an exemplary embodiment of the present disclosure, the behavior described in the simulation logic program may be directly intervened. For example, a user input for modifying the behavior in the simulation logic program may be received. Then, in step 502, at least one of the rule, the parameter and the data may be modified according to the user input. Subsequently, in step 503, the modified at least one of the rule, the parameter and the data may be applied to the preset simulation logic program and the preset simulation logic program is run to obtain and output the simulation result data.

Furthermore, according to an exemplary embodiment of the present disclosure, it is possible to directly intervene a change in the state described in the simulation logic program. For example, a user input for modifying the state described in the simulation logic program may be received. Subsequently, a change in at least one of the state may be modified based on the user input.

Furthermore, according to an exemplary embodiment of the present disclosure, the simulation system 100 or the above-described system may be initialized. For example, a user input for initialization may be received. Then, an initial value of the state described in the simulation logic program and a value of the learnable parameter may be set according to the user input. In addition, when the initial value of the state in the simulation logic program cannot be directly obtained from the domain knowledge, the data or the event, the initial value of the state in the simulation logic program may be used as the learnable parameter.

Furthermore, according to an exemplary embodiment of the present disclosure, the learnable parameter may be optimized. For example, reference data for the simulated item may be received. Then, in step 504, the output simulation result data is compared with the reference data of the simulated item, and based on the comparison result, the learnable parameter is optimized by an optimization algorithm.

Furthermore, according to an exemplary embodiment of the present disclosure, after the initialization is completed, when the behavior in the simulation logic program is controlled based on the external input (e.g., at least one of the domain knowledge, the data, the event and the learnable parameter), the simulation logic program may be run such that the behavior in the simulation logic program drives the state in the simulation logic program to change from an initial state to a next state, so that the changed state may be output as the simulation result data. When the external input includes the learnable parameter, the value of the learnable parameter may be optimized based on the simulation result data output by the simulation module 103. When a next simulation is performed, an external input may be re-received(for example, at least one of domain knowledge, data, and an event may be re-received), and the optimized value of the learnable parameter after the last optimization may be obtained, and the behavior in the simulation logic program is controlled based on the re-received external input by using the optimized value as the value of the learnable parameter. Then, based on this control, the simulation logic program may be run again to output the simulation result data, and the current value of the learnable parameter may be optimized again based on the simulation result data. So on and so forth.

That is, in the case where the external input includes the learnable parameter, an operation mode of the simulation system 100 or the above-described system may include an optimization mode and a simulation mode. In the optimization mode, the receiving step, the controlling step, the running step, and the optimizing step are iteratively performed to obtain a final optimized value of the learnable parameter. In the simulation mode, the step of controlling the behavior described in the preset simulation logic program based on an external input may be performed by taking the final optimized value of the learnable parameter as the value of the learnable parameter, and the preset simulation logic program may be run in response to the controlling, and the accurate simulation result data is output.

In addition, according to an exemplary embodiment of the present disclosure , the external input may be received in real time; the behavior described in the preset simulation logic program may be controlled based on the real-time external input; and the preset simulation logic program may be run in real time in response to the control of the control module 102; the value of the learnable parameter may be optimized based on the simulation result data output by the simulation module in real time. For example, the receiving step, the controlling step, the running step, and the optimizing step may be performed iteratively at predetermined time intervals. For another example, the receiving step, the controlling step, and the running step may be performed at a first predetermined time interval, while the optimizing step may be performed at a second predetermined time interval, wherein the second predetermined time interval is longer than the first predetermined time interval. For example, in an application scenario of epidemic deduction, the receiving step, the controlling step and the running step may be executed every day, and the epidemic simulation result data may be output every day, while the optimizing step may be executed every ten days, that is, the learnable parameter may be optimized based on the ten-day epidemic simulation result data, which will be described in detail later.

Furthermore, according to an exemplary embodiment of the present disclosure, a control input may be received, and a corresponding control may be performed according to the control input. For example, the control input includes at least one of a start simulation input for starting a simulation, a simulation intervention input for intervening a simulation, a pause (or end) simulation input for pausing (or ending) a simulation, a data import input for importing data, a switch mode input for switching an optimization mode and a simulation mode, and a result display input for displaying a result, but not limited to this.

Furthermore, according to an exemplary embodiment of the present disclosure, a user interface may be displayed. For example, the user interface may include a button for receiving a control input, received data, and simulation related data. The received data may include any input data such as the external input, the user input for modifying the behavior or the state, the initial value of the state and the value of the learnable parameter as described above, etc. The simulation related data may include at least one of a current mode (e.g., the optimization mode or the simulation mode), the simulation result data, and the reference data of the simulated item.

Hereinafter, embodiments in which the simulation system 100 and the simulation method according to the present disclosure are applied to a vehicle speed simulation scenario and an epidemic deduction simulation scenario will be described in detail.

**The vehicle speed simulation scenario**

The simulation system 100 according to the exemplary embodiment of the present disclosure may be applied as a vehicle speed simulation system 100. The vehicle speed simulation system 100 is used to simulate a change in a vehicle speed. Therefore, in a simulation logic program of the vehicle speed simulation system 100, the vehicle speed v may be set as a state, and an acceleration of the vehicle may be set as a behavior that drives a change in the state. A time period of the simulation (a time interval between two adjacent states) may be set to t.

The user interface module 101 of the vehicle speed simulation system 100 may receive an external input, where the external input may include at least one of domain knowledge, data, and an event related to the change in the vehicle speed.

The control module 102 of the vehicle speed simulation system 100 may control the behavior described in the simulation logic program based on the external input. For example, the control module 102 may convert the domain knowledge related to the change in the vehicle speed into a rule involved in an acceleration behavior and a parameter involved in the acceleration behavior, i.e., an acceleration formula, the speed at a next moment $$v' = v + \frac{F}{m} \times t,$$ where F is the traction force of the vehicle and m is the mass of the vehicle, and F and m are parameters involved in the acceleration behavior.

In addition, when the vehicle is loaded with people or goods, total mass of the vehicle is equal to a sum of the mass of the vehicle itself and the mass of the people and/or goods loaded in the vehicle. Accordingly, the data related to the change in the vehicle speed in the external input may include data related to the goods loaded by the vehicle, such as a type, quantity and unit mass of the goods. The control module 102 may use the data related to the goods loaded by the vehicle to calculate the total mass of the vehicle.

In the above domain knowledge and data related to the change in the vehicle speed, it is impossible to directly obtain a specific symbol of a calculation symbol (for example, sign "+") in the above acceleration formula, so the calculation symbol may be set as a learnable parameter, The optimizing may be performed through the optimization module 104.

In addition, since the traction force F of the vehicle may not be directly obtained due to some factors (e.g., the age of the vehicle), the traction force F of the vehicle may be set as a learnable parameter, and the optimizing may be performed by the optimization module 104.

In addition, the type and quantity of the goods loaded by the vehicle can usually be obtained, but the unit mass of the goods cannot be directly obtained. Therefore, the unit mass of the goods may be set as a learnable parameter, and the optimizing may be performed by the optimization module 104.

In addition, when a user input for modifying the acceleration behavior is received through the user interface module 101, the control module 102 can directly adjust at least one of the rule, the parameter and the quantity involved in the acceleration behavior according to the user input. For example, the user can modify the acceleration formula through the user interface module 101, so the control module 102 can modify the acceleration formula in the simulation logic program according to the user input.

In addition, when a user input for modifying the vehicle speed or an emergency (e.g., there are pedestrians on a driving path) is received through the user interface module 101, the control module 102 can directly adjust a change in the vehicle speed according to the user input or the emergency, for example, directly modify a next state of the vehicle speed to 0.

In the above example, the vehicle speed simulation system 100 involves three learnable parameters, that is, (1) the calculation symbol in the acceleration formula, whose type is a string, and the value range is four arithmetic symbols "+, -, $\times$, $\div$"; (2) the vehicle traction F, whose type is a floating-point number, and the value range is [0,10000] Newton; (3) the unit mass of goods, whose type is a floating-point number, whose value range is [0,1000] Kilogram.

The optimization module 104 may perform optimizing on the above three learnable parameters. Specifically, in an initialization stage of the vehicle speed simulation system 100, an initial value of the vehicle speed state and values of the three learnable parameters may be set. Under the control of the control module 102, the change in the vehicle speed state after a predetermined time interval t(e.g., 10 seconds) is simulated by the simulation module 103 and the next state of the simulated vehicle speed is output (e.g., after 10 seconds, the vehicle speed is accelerated from the initial speed of 0 to v m/s). Assuming that there is observation data of a real system, for example, in the real world, the vehicle carries the goods, and after 10 seconds of acceleration, the vehicle speed is accelerated from the initial speed of 0 to 10 m/s. Therefore, the optimization module 104 can compare the simulation result data v with the real reference data 10m/s, for example, calculate a simulation error as an optimization target, such as $(v - 10)^2$. By tuning the above three learnable parameters to minimize the optimization target, the purpose of optimizing the above three learnable parameters is achieved.

In addition, in the above example, considering that the above three learnable parameters include multiple types, the optimization module 104 can use a highly versatile evolutionary algorithm to optimize the above three learnable parameters, for example, can find one or more sets of learnable parameters that satisfies v = 10m/s.

In the vehicle speed simulation system 100, the vehicle speed may be simulated every predetermined time interval t, and the learnable parameters may be optimized once. Alternatively, the vehicle speed may be simulated every predetermined time interval t, and the learnable parameters may be optimized once with ten times of simulation results every predetermined time interval 10t. For example, a mean squared error between the ten times of simulation results and real data may be calculated to optimize the learnable parameters.

**The epidemic deduction simulation scene**

The simulation system 100 according to the exemplary embodiment of the present disclosure may be applied as an epidemic game simulation system 100. The epidemic deduction simulation system 100 is used to simulate development of an epidemic. For example, the epidemic deduction simulation system 100 may perform a simulation of the development of the epidemic in a predetermined area (e.g., one of a world, a continent, a country, a province, a city, a district, and a community), in a predetermined simulation time period (e.g., one of day, month, and year).

As shown in FIG. 6, FIG. 6 is a schematic diagram illustrating an epidemic deduction simulation logic according to an exemplary embodiment of the present disclosure. The simulation logic in FIG. 6 is applicable to each province, city, district, and community, as well as for each day in a simulation process. Hereinafter, a city is taken as an example to be described, but it is not limited to this. In FIG. 6, the dotted box represents a boundary of the city, the solid boxes represent states involved in the simulation logic, and the arrows represent behaviors involved in the simulation logic. Among them, moving-in infected population and moving-in normal population are the sum of all corresponding populations who migrated to the city (from different cities).

Specifically, in the simulation logic program of the epidemic deduction simulation system 100, a state related to the development of the epidemic may be set as the state, and a behavior related to the development of the epidemic may be set as the behavior that drives the change in the state. According to an exemplary embodiment of the present disclosure, the state related to the development of the epidemic may include at least one of a number of normal population, a number of moving-in/moving-out normal population, a number of moving-in/moving-out infected population, a number of incubation patients, a number of disease patients, a number of quarantined patients, a number of confirmed patients, a number of dead patients, and a number of cured patients, within a predetermined area (such as a city). The behavior related to the development of the epidemic may include at least one of an internal infection behavior, a migration infection behavior, a disease behavior, an quarantined behavior, a confirmed behavior, a dead behavior, a cured behavior, a moving-in behavior and a moving-out behavior.

The user interface module 101 of the epidemic deduction simulation system 100 may receive an external input, where the external input may include at least one of domain knowledge, data, and an event related to the development of the epidemic.

The control module 102 of the epidemic deduction simulation system 100 can control the behavior described in the simulation logic program based on the external input. For example, the control module 102 may convert the external input into a rule (e.g., the formula for the state change as described above), a parameter (e.g., at least one of a disease infectivity capability value, an internal infectivity correction coefficient for the predetermined area, a migration infectivity correction coefficient for the predetermined area, a proportion of disease patients, an incubation period time, a probability distribution of incubation patients, a disease characteristic and a medical resource) and data (e.g., at least one of internal population flow data, the total number of moving-in population and the total number of moving-out population) involved in the behavior as described above. Furthermore, in accordance with an exemplary embodiment of the present disclosure, the control module 102 may include a plurality of controllers, each controller handling one behavior, and may also include a controller for modifying the behavior and/or a controller for modifying the state.

Specifically, according to an exemplary embodiment of the present disclosure, for the moving-in behavior, that is, the behavior of the normal population and the infected population moving into the city, the control module 102 may set a rule involved in the moving-in behavior as:

the number of new moving-in normal population = F1 (a total number of moving-in population; a proportion of disease patients),
the number of new moving-in infected population = F2 (a total number of moving-in population; a proportion of disease patients),
wherein F1() and F2() are rule functions of the moving-in behavior, the total number of moving-in population is data involved in the rule functions of the moving-in behavior, and the proportion of disease patients is a parameter involved in the rule functions of the moving-in behavior. Here, the total number of moving-in population may be real data, and the proportion of disease patients may be obtained through calculation, for example, the proportion of disease patients = (the number of incubation patients + the number of disease patients) / (the number of normal population + the number of incubation patients + the number of disease patients). In addition, for example, the number of new moving-in infected population = the total number of moving-in population × the proportion of disease patients, and the number of new moving-in normal population = the total number of moving-in population × (1 - the proportion of disease patients) , but not limited to this. This application does not limit the specific content of F1() and F2().

In addition, according to an exemplary embodiment of the present disclosure, for the moving-out behavior, that is, the behavior of the normal population and the infected population moving out of the city, the control module 102 may set a rule involved in the moving-out behavior as:

the number of new moving-out normal population = F3 (the number of normal population, the number of incubation patients, the number of disease patients, a total number of moving-out population; a proportion of disease patients),
the number of new moving-out infected population = F4 (the number of normal population, the number of incubation patients, the number of disease patients, the total number of moving-out population; a proportion of disease patients),
wherein F3() and F4() are rule functions of the moving-out behavior. The number of normal population, the number of incubation patients and the number of disease patients are states involved in the rule functions of the moving-out behavior, and the total number of moving-out population is data involved in the rule functions of the moving-out behavior, the proportion of disease patients is a parameter involved in the rule functions of the moving-out behavior. Here, the total number of moving-out population may be real data, and the proportion of disease patients may be obtained through calculation, for example, the proportion of disease patients = (the number of incubation patients + the number of disease patients) / (the number of normal population + the number of incubation patients + the number of disease patients). In addition, for example, the number of new moving-out infected population = the total number of moving-out population × the proportion of disease patients, and the number of new moving-out normal population = the total number of moving-out population × (1 - the proportion of disease patients) , but not limited to this. This application does not limit the specific content of F3() and F4().

In addition, according to an exemplary embodiment of the present disclosure, for the internal infection behavior, that is, the behavior of the increase of incubation patients due to the normal population contact with the incubation patients and the disease patients in the city, the control module 102 can set a rule involved in the internal infection behavior as:

the number of new incubation patients = F5 (the number of normal population, the number of disease patients, the number of incubation patients, the internal population flow data; the disease infectivity capability value, the internal infectivity correction coefficient for the predetermined area),
wherein F5() is a rule function of the internal infection behavior. The number of normal population, the number of disease patients and the number of incubation patients are states involved in the rule function of the internal infection behavior, and the internal population flow data is data involved in the rule function of the internal infection behavior, the disease infectivity capability value and the internal infectivity correction coefficient for the predetermined area are parameters involved in the rule function of the internal infection behavior. Here, the internal population flow data may refer to population flow data within the city, the disease infectivity capability value may refer to a general infectivity capability value of a disease, and the internal infectivity correction coefficient may refer to a value for correcting the internal infectivity of the disease within the predetermined area (because the infectivity of the disease may be different in different areas), that is, the internal infectivity capability value of the disease within the predetermined area may correspond to a product of the infectivity capability value of the disease and the internal infectivity correction coefficient for the predetermined area (e.g., a city). The internal population flow data may be real data, and the disease infectivity capability value and the internal infectivity correction coefficient for the predetermined area (e.g., a city) may be learnable parameters because they cannot be directly obtained. This application does not limit the specific content of F5().

In addition, according to an exemplary embodiment of the present disclosure, for the migration infection behavior, that is, the behavior of the increase of incubation patients due to the moving-in normal population contact with the moving-in infected population (including the incubation patients and the disease patients), the control module 102 may set a rule involved in the migration infection behavior as:

the number of new incubation patients = F6 (the number of moving-in normal population, the number of moving-in infected population; the disease infectivity capability value, the migration infectivity correction coefficient for the predetermined area),
wherein F6() is a rule function of the migration infection behavior. The number of moving-in normal population and the number of moving-in infected population are states involved in the rule function of the migration infection behavior, the disease infectivity capability value and the migration infectivity correction coefficient for the predetermined area are parameters involved in the rule function of the migration infection behavior. Here, the disease infectivity capability value may refer to a general infectivity capability value of a disease, and the migration infectivity correction coefficient may refer to a value for correcting the migration infectivity of the disease within the predetermined area (because the infectivity of the disease during migration may be different), that is, the migration infectivity capability value of the disease within the predetermined area may correspond to a product of the infectivity capability value of the disease and the migration infectivity correction coefficient for the predetermined area (e.g., a city). The disease infectivity capability value and the migration infectivity correction coefficient for the predetermined area (e.g., a city) may be learnable parameters because they cannot be directly obtained. This application does not limit the specific content of F6().

In addition, according to an exemplary embodiment of the present disclosure, for the disease behavior, that is, the behavior of the patient in the incubation period transitioning from the incubation period to the disease patient, the control module 102 may set a rule involved in the disease behavior as:

the number of new disease patients = F7 (the number of incubation patients; the incubation period time, a probability distribution of disease patients),
wherein F7() is a rule function of the disease behavior. The number of incubation patients is a state involved in the rule function of the disease behavior, and the incubation period time and the probability distribution of the disease patients are parameters involved in the rule function of the disease behavior.

Here, the incubation period time and the probability distribution of disease patients may be real data or values calculated based on the real data. This application does not limit the specific content of F7 ().

In addition, according to an exemplary embodiment of the present disclosure, for the quarantined behavior, that is, the behavior that the disease patient is quarantined, the control module 102 may set a rule involved in the quarantined behavior as:

the number of new quarantined patients = F8 (the number of new disease patients; the medical resource),
wherein F8() is a rule function of the quarantined behavior. The number of new disease patients is a state involved in the rule function of the quarantined behavior, and the medical resource is a parameter involved in the rule function of the quarantined behavior. Here, considering that a part of new quarantined patients cannot be quarantined due to insufficient medical resources, the medical resource may be used as a parameter involved in the rule function of the quarantined behavior, wherein the medical resource may be obtained from real data. This application does not limit the specific content of F 8 ( ).

In addition, according to an exemplary embodiment of the present disclosure, for the confirmed behavior, that is, the behavior of the quarantined patient being confirmed, the control module 102 may set a rule involved in the confirmed behavior as:

the number of new confirmed patients = F9 (the number of quarantined patients; the disease characteristic, the medical resource),
wherein F9() is a rule function of the confirmed behavior. The number of quarantined patients is a state involved in the rule function of the confirmed behavior, and the disease characteristics and the medical resource are parameters involved in the rule function of the confirmed behavior. Here, the disease characteristic may be obtained from domain knowledge or prior knowledge, or may be set as a learnable parameter if it cannot be obtained directly, and the medical resource may be obtained from real data. This application does not limit the specific content of F9().

In addition, according to an exemplary embodiment of the present disclosure, for the dead behavior, that is, the behavior of the confirmed patient being dead, the control module 102 may set a rule involved in the dead behavior as:

the number of new dead patients = F10 (the number of confirmed patients; the disease characteristic, the medical resource),

wherein F10() is a rule function of the dead behavior. The number of confirmed patients is a state involved in the rule function of the dead behavior, and the disease characteristic and the medical resource are parameters involved in the rule function of the dead behavior. Here, the disease characteristic may be obtained from domain knowledge or prior knowledge, or may be set as a learnable parameter if it cannot be obtained directly, and the medical resource may be obtained from real data. This application does not limit the specific content of F10().

In addition, according to an exemplary embodiment of the present disclosure, for the cured behavior, that is, the behavior of the confirmed patient being cured, the control module 102 may set a rule involved in the cured behavior as:

the number of new cured patients = F11 (the number of confirmed patients; the disease characteristics, the medical resource),
wherein F11() is a rule function of the cured behavior. The number of confirmed patients is a state involved in the rule function of the cured behavior, and the disease characteristic and the medical resource are parameters involved in the rule function of the cured behavior. Here, the disease characteristic may be obtained from domain knowledge or prior knowledge, or may be set as a learnable parameter if it cannot be obtained directly, and the medical resource may be obtained from real data . This application does not limit the specific content of F11().

In addition, according to an exemplary embodiment of the present disclosure, the control module 102 may further set a behavior rule that drives changes of the states described above based on the above behavior rule:

the number of normal population = the number of normal population + the number of new normal population = the number of normal population + the number of new moving-in normal population - the number of new moving-out normal population + the number of new cured patients;
the number of moving-in normal population = the number of new moving-in normal population;
the number of moving-out normal population = the number of new moving-out normal population;
the number of moving-in infected population = the number of new moving-in infected population;
the number of moving-out infected population = the number of new moving-out population;
the number of incubation patients = the number of incubation patients + the number of new incubation patients - the number of new disease patients + the number of moving-in infected population - the number of moving-out infected population, wherein the number of new incubation patients = the number of incubation patients for the internal infection behavior + the number of incubation patients for the migration infection behavior;

the number of disease patients = the number of disease patients + the number of new disease patients - the number of new quarantined patients;

the number of quarantined patients = the number of quarantined patients + the number of new quarantined patients - the number of new confirmed patients;

the number of confirmed patients = the number of confirmed patients + the number of new confirmed patients - the number of new dead patients - the number of new cured patients;

the number of dead patients = the number of dead patients + the number of new dead patients;

the number of cured patients = the number of cured patients + the number of new cured patients.

Of course, the above states and behaviors of the development of the epidemic, as well as the rules, parameters and data involved in the behaviors are an exemplary, the simulation logic program can also set any other states and behaviors according to user requirements, and the control module 102 can also set any other rules, parameters and data.

Subsequently, the simulation module 103 may run the simulation logic program in response to the above control of the control module 104, and output the epidemic simulation result.

In addition, when a user input for modifying the above behavior is received through the user interface module 101, the control module 102 can directly adjust at least one of the rule, parameter and data involved in the above behavior according to the user input. For example, the user can modify a rule function related to the migration infection behavior through the user interface module 101, so the control module 102 can modify the rule function related to the migration infection behavior in the simulation logic program according to the user input.

In addition, when a user input for modifying the above state or an emergency (for example, a concentrated outbreak of disease in a prison) is received through the user interface module 101, the control module 102 can directly modify a change in the state according to the user input or the emergency. For example, the number of new disease patients may be directly modified to 200.

In the above domain knowledge and data related to the development of the epidemic, it is impossible to directly obtain at least one of the disease infectivity capability value, the initial value of the number of incubation patients in the predetermined area, the internal infectivity correction coefficient for the predetermined area, the migration infectivity correction coefficient for the predetermined area range, and the disease characteristic involved in the above rule, therefore, the at least one of the disease infectivity capability value, the initial value of the number of incubation patients in the predetermined area, the internal infectivity correction coefficient for the predetermined area, the migration infectivity correction coefficient for the predetermined area range, and the disease characteristic is set as a learnable parameter, and may be optimized by the optimization module 104.

Specifically, in the initialization stage of the epidemic deduction simulation system 100, the initial value of the state as described above and the value of the learnable parameter as described above may be received through the user interface module 101. For example, the user can set the initial value of the normal population to the total population within the predetermined area; set the initial values of the number of moving-in/moving-out normal population, the number of moving-in/moving-out infected population, the number of incubation patients, the number of disease patients, the number of quarantined patients, the number of confirmed patients, the number of dead patients and the number of cured patients based on an epidemic start date and a simulation start date. For example, the initial value of the number of incubation patients may be set to a non-zero value and the initial values of the number of moving-in/moving-out normal population, the number of moving-in/moving-out infected population, the number of disease patients, the number of quarantined patients, the number of confirmed patients, the number of dead patients and the number of cured patients may be set to zero, based on the epidemic start date and the simulation start date. The user can set a value of at least one of the disease infectivity capability value, the internal infectivity correction coefficient for the predetermined area, the migration infectivity correction coefficient for the predetermined area, and the disease characteristic according to domain knowledge and prior knowledge.

Under the control of the control module 102, the simulation module 103 simulates changes in the states as described above after a predetermined time interval t (e.g., one day), and outputs simulated epidemic data. In addition, reference data about the development of the epidemic, that is, real epidemic data of the current day may be received through the user interface module 101. Therefore, the optimization module 104 can compare the simulated epidemic data with the real epidemic data, for example, calculate a simulation error as an optimization target, and minimize the optimization target by adjusting the learnable parameter as described above, so as to achieve the purpose for optimizing the above learnable parameter.

In the epidemic deduction simulation system 100, in the optimization mode, the development of the epidemic may be simulated once a day by using the daily updated external input, and the learnable parameter may be optimized once a day. Alternatively, the development of the epidemic may be simulated once a day by using the daily updated external input, and the learnable parameter is optimized once every predetermined number of days (e.g., ten days) by using the simulation results for the predetermined number of days. For example, the simulated epidemic data may be the simulated number of confirmed patients within the predetermined area that is output every day, and the real epidemic data may be the actual number of confirmed patients within the predetermined area every day. The optimization module 104 may calculate a mean squared error between the simulated number of confirmed patients and the actual number of confirmed patients for the predetermined number of days (e.g., ten days), and perform optimizing the learnable parameter through an evolutionary algorithm based on the calculated mean squared error.

In the epidemic deduction simulation system 100, in the simulation mode, the development of the epidemic may be simulated once a day by using the external input updated every day and the learnable parameter that is finally optimized.

In addition, in the epidemic deduction simulation system 100, the user interface module 101 may receive a control input. For example, the control input includes at least one of a start button for starting a simulation, a intervention button for intervening a simulation, a pause button for pausing (or ending) a simulation, an import button for importing data, a switch button for switching an optimization mode and a simulation mode and a display button for displaying a result, but not limited to this. The control module 102 may perform a corresponding control according to the control input.

In addition, in the epidemic deduction simulation system 100, the user interface module 101 may also be used as a display module for displaying a user interface under the control of the control module 102. The user interface module 101 may also be implemented by a touch-sensitive screen. The user interface displayed by the user interface module 101 may include a button for receiving the control input, data received by the user interface module 101, and simulation-related data. Here, the data received by the user interface module 101 may include any input data such as the external input, the user input for modifying the behavior or state, the initial value of the state and the value of the learnable parameter as described above, and the like. The simulation-related data may include at least one of a current mode, simulation result data, and reference data of the epidemic development.

The data management module 105 may store and manage data related to the epidemic deduction simulation system 100. For example, these data may include preset data, real-time data, and user data. Here, the preset data may refer to data used by the developer when developing the epidemic deduction simulation system 100, which may be provided to the customer together with the epidemic deduction simulation system 100. The real-time data may refer to data that changes with time, which is continuously updated during the use of the epidemic deduction simulation system 100. The user data may refer to data provided by a user. These data may be received through the user interface module 101, or may be pre-stored, or may be transmitted and received through a network, etc., which is not limited in the disclosure. The data management interface module 106 may import at least one piece of data in the data management module 105 into the control module 102. For example, the data management module 105 may store and manage the external input received through the user interface module 101 and import the external input to the control module 102 through the data management interface module 106 in response to, for example, a control input through the user interface module 101 .

The computation and storage engine module 107 may support computing and storage requirements of the epidemic deduction simulation system 100. The computation and storage engine module 107 may provide the epidemic deduction simulation system 100 with underlying resources, including computing resources and storage resources. The computing resources may include algorithm libraries, computing frameworks, computing hardware, and the like. The storage resources may include database software, memory hardware, distributed storage servers, and the Internet and so on.

The simulation system and the simulation method according to the exemplary embodiments of the present disclosure have been described above with reference to FIGS. 1 to 6 .

The systems, devices, and units illustrated in FIG. 1 may be respectively configured as software, hardware, firmware, or any combination thereof to execute specific functions. For example, these systems, devices, or units may correspond to dedicated integrated circuits, may also correspond to pure software codes, or may correspond to modules combining software and hardware. In addition, one or more functions implemented by these systems, devices, or units may also be uniformly executed by components in a physical entity device (for example, a processor, a client, or a server, etc.).

In addition, the method described with reference to FIG. 5 may be implemented by a program (or instruction) recorded on a computer-readable storage medium.

For example, according to the exemplary embodiment of the present disclosure, a computer-readable storage medium for simulating may be provided, wherein the computer-readable storage medium has a computer program (or instruction) for executing the simulation method described with reference to FIG. 5 recorded thereon. For example, the computer program (or instruction) may be used to execute the following method steps: receiving an external input; controlling a behavior described in a preset simulation logic program based on the external input, wherein the preset simulation logic program describes a state in a simulated item and a behavior that drives a change in the state in code, wherein when the external input includes a learnable parameter, an optimized value of the learnable parameter is obtained, and the step of controlling the behavior described in the preset simulation logic program based on the external input is performed by using the optimized value as a value of the learnable parameter; in response to the controlling, running the preset simulation logic program; optimizing the value of the learnable parameter based on output simulation result data.

For another example, according to the exemplary embodiment of the present disclosure, a computer-readable storage medium for epidemic deduction simulation may be provided, wherein the computer-readable storage medium has a computer program (or instruction) for executing a simulation method in an epidemic deduction scenario recorded thereon. For example, the simulation method may include: receiving an external input; controlling a behavior described in a preset simulation logic program based on the external input, wherein the preset simulation logic program describes a state in development of an epidemic and a behavior that drives a change in the state in the development of the epidemic in code, wherein when the external input includes a learnable parameter, an optimized value of the learnable parameter is obtained, and the step of controlling the behavior described in the preset simulation logic program based on the external input is performed by using the optimized value as a value of the learnable parameter; in response to the controlling, running the preset simulation logic program; optimizing the value of the learnable parameter based on output simulation result data.

The computer program in the above computer-readable storage medium may run in an environment deployed in computer devices such as a client, a host, an agent device and a server. It should be noted that the computer program may also be used to execute additional steps in addition to the above steps, or execute more specific processing when executing the above steps. These additional steps and further processed content have been mentioned in the description of the related method with reference to FIGS. 5 and 6, and thus will not be repeated here in order to avoid repetition.

It should be noted that the simulation system according to the exemplary embodiments of the present disclosure may completely rely on the running of the computer program to implement corresponding functions, that is, respective units correspond to respective steps in the functional architecture of the computer program, so that the entire system is called through a special software package (for example, lib library) to implement the corresponding functions.

On the other hand, respective devices illustrated in FIG. 1 may also be implemented by hardware, software, firmware, middleware, microcode, or any combination thereof. When implemented by software, firmware, middleware or microcode, a program code or code segment used to perform a corresponding operation may be stored in a computer-readable storage medium such as a storage medium, so that a processor may read and execute the corresponding program code or code segment to perform the corresponding operation.

For example, the exemplary embodiments of the present disclosure may also be implemented as a computing device, which includes a storage component and a processor. The storage component stores a set of computer-executable instructions, and when the set of computer-executable instructions is executed by the processor, the simulation method according to the exemplary embodiments of the present disclosure is executed.

In particular, the computing device may be deployed in a server or a client, and may also be deployed on a node device in a distributed network environment. In addition, the computing device may be a PC computer, a tablet device, a personal digital assistant, a smart phone, a web application, or other devices capable of executing the above set of instructions.

Here, the computing device does not have to be a single computing device, but may also be an assembly of any device(s) or circuit(s) that may execute the above instructions (or set of instructions) separately or jointly. The computing device may also be a part of an integrated control system or a system manager, or may be configured as a portable electronic device interconnecting with a local or remote (for example, via wireless transmission) interface.

In the computing device, the processor may include a central processing unit (CPU), a graphics processing unit (GPU), a programmable logic device, a dedicated processor system, a microcontroller, or a microprocessor. As an example rather than a limitation, the processor may also include an analog processor, a digital processor, a microprocessor, a multi-core processor, a processor array, a network processor, etc.

Some operations described in the simulation method according to the exemplary embodiments of the present disclosure may be implemented by software, and some operations may be implemented by hardware. In addition, these operations may also be implemented by a combination of software and hardware.

The processor may execute instructions or codes stored in one of the storage components, wherein the storage component may also store data. Instructions and data may also be sent and received via a network through a network interface device, wherein the network interface device may adopt any known transmission protocol.

The storage component may be integrated with the processor, for example, an RAM or flash memory is arranged in an integrated circuit microprocessor, etc. In addition, the storage component may include an independent device, such as an external disk drive, a storage array, or any other storage device that may be used by a database system. The storage component and the processor may be operatively coupled, or may communicate with each other, for example, through an I/O port, a network connection, etc., so that the processor may read files stored in the storage component.

In addition, the computing device may also include a video display (such as a liquid crystal display) and a user interaction interface (such as a keyboard, a mouse, a touch input device, etc.). All components of the computing device may be connected to each other via at least one of a bus and a network.

The operations involved in the simulation method according to the exemplary embodiments of the present disclosure may be described as various interconnected or coupled functional blocks or functional diagrams. However, these functional blocks or functional diagrams may be equally integrated into a single logic device or operate according to imprecise boundaries.

Therefore, a system including at least one computing device and at least one storage device storing instructions is provided. The instructions, when executed by at least one computing device, cause the at least one computing device to perform a simulation method according to the exemplary embodiment of the present disclosure.

According to the exemplary embodiment of the present disclosure, the at least one computing device is a computing device for simulating according to the exemplary embodiment of the present disclosure, and a set of computer-executable instructions is stored in the storage device. When the set of computer-executable instructions is executed by the at least one computing device, the following steps are executed: receiving an external input; controlling a behavior described in a preset simulation logic program based on the external input, wherein the preset simulation logic program describes a state in a simulated item and a behavior that drives a change in the state in code, wherein when the external input includes a learnable parameter, an optimized value of the learnable parameter is obtained, and the step of controlling the behavior described in the preset simulation logic program based on the external input is performed by using the optimized value as a value of the learnable parameter; in response to the controlling, running the preset simulation logic program; optimizing the value of the learnable parameter based on output simulation result data.

In addition, a system including at least one computing device and at least one storage device storing instructions is provided. The instructions, when executed by at least one computing device, cause the at least one computing device to perform a epidemic deduction simulation method according to the exemplary embodiment of the present disclosure.

According to the exemplary embodiment of the present disclosure, the at least one computing device is a computing device for simulating according to the exemplary embodiment of the present disclosure, and a set of computer-executable instructions is stored in the storage device. When the set of computer-executable instructions is executed by the at least one computing device, the epidemic deduction simulation method is executed, including: receiving an external input; controlling a behavior described in a preset simulation logic program based on the external input, wherein the preset simulation logic program describes a state in development of an epidemic and a behavior that drives a change in the state in the development of the epidemic in code, wherein when the external input includes a learnable parameter, an optimized value of the learnable parameter is obtained, and the step of controlling the behavior described in the preset simulation logic program based on the external input is performed by using the optimized value as a value of the learnable parameter; in response to the controlling, running the preset simulation logic program; optimizing the value of the learnable parameter based on output simulation result data.

Various exemplary embodiments of the present disclosure are described above. It should be understood that the above descriptions are an exemplary and not exhaustive. The present disclosure is not limited to the disclosed various exemplary embodiments. With no deviation from the scope and spirit of the present disclosure, many modifications and variations are obvious to those skilled in the art. Therefore, the protection scope of the present disclosure should be subject to the scope of the claims.

Industrial Applicability

According to the simulation system and simulation method of the present disclosure, real data (for example, historical data) can be used to determine parameters required for simulation that cannot be obtained from domain knowledge temporarily by fitting the real data, thereby reducing the dependence of the simulation system on domain knowledge, expanding the applicable field and scope of the simulation system, meeting the simulation requirements of many different scenarios, and improving the simulation accuracy.

In addition, according to the epidemic deduction simulation system and simulation method suitable for epidemic deduction scenarios of the present disclosure, compared with traditional epidemic models (e.g., SIR, SEIR), a more complex epidemic deduction simulation logic can be realized, and real epidemic data can be utilized to determine epidemic deduction parameters required for simulation that cannot be obtained from epidemic domain knowledge temporarily by fitting the real epidemic data, so as to obtain more accurate epidemic deduction simulation results.

**Claims**

1. A system comprising at least one computing device and at least one storing device, wherein the at least one storing device stores instructions that, when executed by the at least one computing device, cause the at least one computing device to perform a simulation method, the simulation method comprises:

receiving an external input;

controlling a behavior described in a preset simulation logic program based on the external input, wherein the preset simulation logic program describes a state in a simulated item and a behavior that drives a change in the state in code, wherein when the external input comprises a learnable parameter, an optimized value of the learnable parameter is obtained, and the step of controlling the behavior described in the preset simulation logic program based on the external input is performed by using the optimized value as a value of the learnable parameter;

in response to the controlling, running the preset simulation logic program;

optimizing the value of the learnable parameter based on output simulation result data.

2. The system of claim 1, wherein when the external input comprises the learnable parameter, an operating mode of the system comprises an optimization mode and a simulation mode;

in the optimization mode, the receiving step, the controlling step, the running step and the optimizing step are iteratively performed, to obtain a final optimized value of the learnable parameter;

in the simulation mode, the step of controlling the behavior described in the preset simulation logic program based on the external input is performed by using the final optimized value of the learnable parameter as the value of the learnable parameter.

3. The system of claim 1, wherein,

the receiving step comprises receiving the external input in real time;

the controlling step comprises controlling the behavior described in the simulation logic program based on the real-time external input;

the running step comprises running the preset simulation logic program in real time in response to the controlling;

the optimizing step comprises optimizing the value of the learnable parameter based on the real-time output simulation result data.

4. The system of claim 1, wherein the external input comprises at least one of domain knowledge, data and an event related to the simulated item.

5. The system of claim 1, wherein the learnable parameter comprises at least one parameter related to the simulated item that is not directly obtainable from domain knowledge, data or an event.

6. The system of claim 1, wherein the behavior involves at least one of a rule, a parameter and data.

7. The system of claim 6, wherein the controlling step comprises:

converting the external input into at least one of the rule, the parameter and the data involved in the behavior,

wherein the running step comprises:

applying the converted at least one of the rule, the parameter and the data to the preset simulation logic program and running the preset simulation logic program, to obtain and output the simulation result data.

8. The system of claim 6, wherein the instructions, when executed by the at least one computing device, cause the at least one computing device to further perform:

receiving a user input for modifying the behavior;

modifying at least one of the rule, the parameter and the data involved in the behavior according to the user input,

wherein the running step comprises:

applying the modified at least one of the rule, the parameter and the data involved in the behavior to the preset simulation logic program and running the preset simulation logic program, to obtain and output the simulation result data.

9. The system of claim 1, wherein the instructions, when executed by the at least one computing device, cause the at least one computing device to further perform:

receiving a user input for initializing the system;

setting an initial value of the state and the value of the learnable parameter according to the user input.

10. The system of claim 1, wherein the instructions, when executed by the at least one computing device, cause the at least one computing device to further perform:

receiving a user input for modifying the state;

modifying a change in at least one of the state based on the user input.

11. The system of claim 1, wherein the instructions, when executed by the at least one computing device, cause the at least one computing device to further perform:

receiving reference data of the simulated item;

wherein the optimizing step comprises:

comparing the output simulation result data with the reference data of the simulated item;

optimizing the learnable parameter by an optimization algorithm, based on a result of the comparison.

12. The system of claim 1, wherein the instructions, when executed by the at least one computing device, cause the at least one computing device to further perform:

receiving a control input;
performing a corresponding control according to the control input;
wherein the control input comprises at least one of a start simulation input, a simulation intervention input, a pause simulation input, a data import input, a switch mode input and a result display input.

13. The system of claim 1, wherein the instructions, when executed by the at least one computing device, cause the at least one computing device to further perform:

displaying a user interface;
wherein the user interface comprises a button for receiving a control input, received data and simulation-related data;
wherein the received data comprises the external input;
wherein the simulation-related data comprises at least one of a current mode, the output simulation result data, and reference data of the simulated item.

14. The system of any of claims 1 to 13, wherein the simulated item is a change in a vehicle speed;

the state is the vehicle speed;
the behavior is acceleration of the vehicle;
the external input comprises at least one of domain knowledge, data and event related to the change in the vehicle speed;
a rule involved in the behavior comprises an acceleration formula, a parameter involved in the behavior comprises a traction force and a mass of the vehicle, and data involved in the behavior comprises a type, quantity and unit mass of goods loaded by the vehicle;
the learnable parameter comprises at least one of a calculation symbol in the acceleration formula, the traction force, and the unit mass of goods loaded by the vehicle.

15. The system of any of claims 1 to 13, wherein the simulated item is development of an epidemic.

16. The system of claim 15, wherein the state comprises at least one of a number of normal population, a number of moving-in/moving-out normal population, a number of moving-in/moving-out infected population, a number of incubation patients, a number of disease patients, a number of quarantined patients, a number of confirmed patients, a number of dead patients, and a number of cured patients, within a predetermined area.

17. The simulation method of claim 16, wherein a range of the predetermined area is one of a world, a continent, a country, a province, a city, a district, and a community.

18. The simulation method of claim 16, wherein the behavior comprises at least one of an internal infection behavior, a migration infection behavior, a disease behavior, an quarantined behavior, a confirmed behavior, a dead behavior, a cured behavior, a moving-in behavior and a moving-out behavior.

19. The system of claim 18, wherein,

a rule involved in the behavior comprises a formula for calculating a change in the state;
a parameter involved in the behavior comprises at least one of a disease infectivity capability value, an internal infectivity correction coefficient for the predetermined area, a migration infectivity correction coefficient for the predetermined area, a proportion of disease patients, an incubation period time, a probability distribution of incubation patients, a disease characteristic and a medical resource;
data involved in the behavior comprises at least one of internal population flow data, the total number of moving-in population and the total number of moving-out population.

20. The system of claim 19, wherein the learnable parameter comprises at least one of a disease infectivity capability value, an initial value of the number of incubation patients within the predetermined area, the internal infectivity correction coefficient for the predetermined area, the migration infectivity correction coefficient for the predetermined area and the disease characteristic.

21. The system of claim 20, wherein the instructions, when executed by the at least one computing device, cause the at least one computing device to further perform:

receiving a user input for initializing the system;
setting an initial value of at least one of the number of normal population, the number of moving-in/moving-out normal population, the number of moving-in/moving-out infected population, the number of incubation patients, the number of disease patients, the number of quarantined patients, the number of confirmed patients, the number of dead patients and the number of cured patients according to the user input;

setting a value of at least one of the disease infectivity capability value, the internal infectivity correction coefficient for the predetermined area, the migration infectivity correction coefficient for the predetermined area and the disease characteristic according to the user input.

22. The system of claim 21, wherein the setting of the initial value comprises:

setting the initial value of the normal population to the total population within the predetermined area;
setting the initial value of the number of incubation patients to a non-zero value based on an epidemic start date and a simulation start date;
setting the initial values of the number of moving-in/moving-out normal population, the number of moving-in/moving-out infected population, the number of disease patients, the number of quarantined patients, the number of confirmed patients, the number of dead patients and the number of cured patients to zero.

23. The system of claim 22, wherein the controlling step comprises at least one of:

for the moving-in behavior, setting a rule involved in the moving-in behavior as:

the number of new moving-in normal population = F1 (a total number of moving-in population; a proportion of disease patients),
the number of new moving-in infected population = F2 (a total number of moving-in population; a proportion of disease patients),
wherein F1() and F2() are rule functions of the moving-in behavior, the total number of moving-in population is data involved in the rule functions of the moving-in behavior, and the proportion of disease patients is a parameter involved in the rule functions of the moving-in behavior;

for the moving-out behavior, setting a rule involved in the moving-out behavior as:

the number of new moving-out normal population = F3 (the number of normal population, the number of incubation patients, the number of disease patients, a total number of moving-out population; a proportion of disease patients),
the number of new moving-out infected population = F4 (the number of normal population, the number of incubation patients, the number of disease patients, the total number of moving-out population; a proportion of disease patients),
wherein F3() and F4() are rule functions of the moving-out behavior, the number of normal population, the number of incubation patients and the number of disease patients are states involved in the rule functions of the moving-out behavior, and the total number of moving-out population is data involved in the rule functions of the moving-out behavior, the proportion of disease patients is a parameter involved in the rule functions of the moving-out behavior;

for the internal infection behavior, setting a rule involved in internal infection behavior as:

the number of new incubation patients = F5 (the number of normal population, the number of disease patients, the number of incubation patients, the internal population flow data; the disease infectivity capability value, the internal infectivity correction coefficient for the predetermined area),
wherein F5() is a rule function of the internal infection behavior, the number of normal population, the number of disease patients and the number of incubation patients are states involved in the rule function of the internal infection behavior, and the internal population flow data is data involved in the rule function of the internal infection behavior, the disease infectivity capability value and the internal infectivity correction coefficient for the predetermined area are parameters involved in the rule function of the internal infection behavior;

for the migration infection behavior, setting a rule involved in the migration infection behavior as:

the number of new incubation patients = F6 (the number of moving-in normal population, the number of moving-in infected population; the disease infectivity capability value, the migration infectivity correction coefficient for the predetermined area),
wherein F6() is a rule function of the migration infection behavior, the number of moving-in normal population and the number of moving-in infected population are states involved in the rule function of the migration infection behavior, the disease infectivity capability value and the migration infectivity correction coefficient for the predetermined area are parameters involved in the rule function of the migration infection behavior;

for the disease behavior, setting a rule involved in the disease behavior as:

the number of new disease patients = F7 (the number of incubation patients; the incubation period time, a probability distribution of disease patients), wherein F7() is a rule function of the disease behavior, the number of incubation patients is a state involved in the rule function of the disease behavior, and the incubation period time and the probability distribution of the disease patients are parameters involved in the rule function of the disease behavior;

for the quarantined behavior, setting a rule involved in the quarantined behavior as:

the number of new quarantined patients = F8 (the number of new disease patients; the medical resource), wherein F8() is a rule function of the quarantined behavior, the number of new disease patients is a state involved in the rule function of the quarantined behavior, and the medical resource is a parameter involved in the rule function of the quarantined behavior;

for the confirmed behavior, setting a rule involved in the confirmed behavior as:

the number of new confirmed patients = F9 (the number of quarantined patients; the disease characteristic, the medical resource), wherein F9() is a rule function of the confirmed behavior, the number of quarantined patients is a state involved in the rule function of the confirmed behavior, and the disease characteristics and the medical resource are parameters involved in the rule function of the confirmed behavior;

for the dead behavior, setting a rule involved in the dead behavior as:

the number of new dead patients = F10 (the number of confirmed patients; the disease characteristic, the medical resource),

wherein F10() is a rule function of the dead behavior, the number of confirmed patients is a state involved in the rule function of the dead behavior, and the disease characteristic and the medical resource are parameters involved in the rule function of the dead behavior;

for the cured behavior, setting a rule involved in the cured behavior as:

the number of new cured patients = F11 (the number of confirmed patients; the disease characteristics, the medical resource), wherein F11() is a rule function of the cured behavior, the number of confirmed patients is a state involved in the rule function of the cured behavior, and the disease characteristic and the medical resource are parameters involved in the rule function of the cured behavior.

24. The system of claim 23, wherein the controlling step comprises setting a rule involved in the behavior that drives the change in the state as:

the number of normal population = the number of normal population + the number of new normal population = the number of normal population + the number of new moving-in normal population - the number of new moving-out normal population + the number of new cured patients; the number of moving-in normal population = the number of new moving-in normal population; the number of moving-out normal population = the number of new moving-out normal population; the number of moving-in infected population = the number of new moving-in infected population; the number of moving-out infected population = the number of new moving-out population; the number of incubation patients = the number of incubation patients + the number of new incubation patients - the number of new disease patients + the number of moving-in infected population - the number of moving-out infected population, wherein the number of new incubation patients = the number of incubation patients for the internal infection behavior + the number of incubation patients for the migration infection behavior; the number of disease patients = the number of disease patients + the number of new disease patients - the number of new quarantined patients; the number of quarantined patients = the number of quarantined patients + the number of new quarantined patients - the number of new confirmed patients;

the number of confirmed patients = the number of confirmed patients + the number of new confirmed patients - the number of new dead patients - the number of new cured patients;

the number of dead patients = the number of dead patients + the number of new dead patients;

the number of cured patients = the number of cured patients + the number of new cured patients.

25. The system of claim 16, wherein the receiving step, the controlling step and the running step are performed in units of days;

wherein the simulation result data is the number of simulated confirmed patients in the predetermined area output every day, and the reference data is the number of real confirmed patients in the predetermined area every day;

the optimizing step comprises calculating a mean square error between the number of simulated confirmed patients and the number of real confirmed patients for a predetermined number of days, and optimizing the learnable parameters by using an evolutionary algorithm based on the calculated mean square error.

26. A simulation method performed by a system comprising at least one computing device and at least one storing device, the at least one storing device stores instructions that, when executed by the at least one computing device, cause the at least one computing device to perform the simulation method, the simulation method comprises:

receiving an external input;

controlling a behavior described in a preset simulation logic program based on the external input, wherein the preset simulation logic program describes a state in a simulated item and a behavior that drives a change in the state in code, wherein when the external input comprises a learnable parameter, an optimized value of the learnable parameter is obtained, and the step of controlling the behavior described in the preset simulation logic program based on the external input is performed by using the optimized value as a value of the learnable parameter;

in response to the controlling, running the preset simulation logic program;

optimizing the value of the learnable parameter based on output simulation result data.

27. The simulation method of claim 26, wherein when the external input comprises the learnable parameter, an operating mode of the system comprises an optimization mode and a simulation mode;

in the optimization mode, the receiving step, the controlling step, the running step and the optimizing step are iteratively performed, to obtain a final optimized value of the learnable parameter;

in the simulation mode, the step of controlling the behavior described in the preset simulation logic program based on the external input is performed by using the final optimized value of the learnable parameter as the value of the learnable parameter.

28. The simulation method of claim 26, wherein,

the receiving step comprises receiving the external input in real time;

the controlling step comprises controlling the behavior described in the simulation logic program based on the real-time external input;

the running step comprises running the preset simulation logic program in real time in response to the controlling;

the optimizing step comprises optimizing the value of the learnable parameter based on the real-time output simulation result data.

29. The simulation method of claim 26, wherein the external input comprises at least one of domain knowledge, data and an event related to the simulated item.

30. The simulation method of claim 26, wherein the learnable parameter comprises at least one parameter related to the simulated item that is not directly obtainable from domain knowledge, data or an event.

31. The simulation method of claim 26, wherein the behavior involves at least one of a rule, a parameter and data.

32. The simulation method of claim 26, wherein the controlling step comprises:

converting the external input into at least one of the rule, the parameter and the data involved in the behavior,

wherein the running step comprises:

applying the converted at least one of the rule, the parameter and the data to the preset simulation logic program and running the preset simulation logic program, to obtain and output the simulation result data.

33. The simulation method of claim 32, further comprising:

receiving a user input for modifying the behavior;
modifying at least one of the rule, the parameter and the data involved in the behavior according to the user input,
wherein the running step comprises:
applying the modified at least one of the rule, the parameter and the data involved in the behavior to the preset simulation logic program and running the preset simulation logic program, to obtain and output the simulation result data.

34. The simulation method of claim 26, further comprising:

receiving a user input for initializing the system;
setting an initial value of the state and the value of the learnable parameter according to the user input.

35. The simulation method of claim 26, further comprising:

receiving a user input for modifying the state;
modifying a change in at least one of the state based on the user input.

36. The simulation method of claim 26, further comprising:

receiving reference data of the simulated item;
wherein the optimizing step comprises:

comparing the output simulation result data with the reference data of the simulated item;
optimizing the learnable parameter by an optimization algorithm, based on a result of the comparison.

37. The simulation method of claim 26, further comprising:

receiving a control input;
performing a corresponding control according to the control input;
wherein the control input comprises at least one of a start simulation input, a simulation intervention input, a pause simulation input, a data import input, a switch mode input and a result display input.

38. The simulation method of claim 26, further comprising:

displaying a user interface;
wherein the user interface comprises a button for receiving a control input, received data and simulation-related data;
wherein the received data comprises the external input;
wherein the simulation-related data comprises at least one of a current mode, the output simulation result data, and reference data of the simulated item.

39. The simulation method of any of claims 26 to 38, wherein the simulated item is a change in a vehicle speed;

the state is the vehicle speed;
the behavior is acceleration of the vehicle;
the external input comprises at least one of domain knowledge, data and event related to the change in the vehicle speed;
a rule involved in the behavior comprises an acceleration formula, a parameter involved in the behavior comprises a traction force and a mass of the vehicle, and data involved in the behavior comprises a type, quantity and unit mass of goods loaded by the vehicle;
the learnable parameter comprises at least one of a calculation symbol in the acceleration formula, the traction force, and the unit mass of goods loaded by the vehicle.

40. The simulation method of any of claims 26 to 38, wherein the simulated item is development of an epidemic.

41. The simulation method of claim 40, wherein the state comprises at least one of a number of normal population, a number of moving-in/moving-out normal population, a number of moving-in/moving-out infected population, a number of incubation patients, a number of disease patients, a number of quarantined patients, a number of confirmed patients, a number of dead patients, and a number of cured patients, within a predetermined area.

42. The simulation method of claim 41, wherein a range of the predetermined area is one of a world, a continent, a country, a province, a city, a district, and a community.

43. The simulation method of claim 41 wherein the behavior comprises at least one of an internal infection behavior, a migration infection behavior, a disease behavior, an quarantined behavior, a confirmed behavior, a dead behavior, a cured behavior, a moving-in behavior and a moving-out behavior.

44. The simulation method of claim 43, wherein,

a rule involved in the behavior comprises a formula for calculating a change in the state;
a parameter involved in the behavior comprises at least one of a disease infectivitycapability value, an internal infectivity correction coefficient for the predetermined area, a migration infectivity correction coefficient for the predetermined area, a proportion of disease patients, an incubation period time, a probability distribution of incubation patients, a disease characteristic and a medical resource;
data involved in the behavior comprises at least one of internal population flow data, the total number of moving-in population and the total number of moving-out population.

45. The simulation method of claim 44, wherein the learnable parameter comprises at least one of a disease infectivitycapability value, an initial value of the number of incubation patients within the predetermined area, the internal infectivity correction coefficient for the predetermined area, the migration infectivity correction coefficient for the predetermined area and the disease characteristic.

46. The simulation method of claim 45, further comprising:

receiving a user input for initializing the system; setting an initial value of at least one of the number of normal population, the number of moving-in/moving-out normal population, the number of moving-in/ moving-out infected population, the number of incubation patients, the number of disease patients, the number of quarantined patients, the number of confirmed patients, the number of dead patients and the number of cured patients according to the user input;
setting a value of at least one of the disease infectivity capability value, the internal infectivity correction coefficient for the predetermined area, the migration infectivity correction coefficient for the predetermined area and the disease characteristic according to the user input.

47. The simulation method of claim 46, wherein the setting of the initial value comprises:

setting the initial value of the normal population to the total population within the predetermined area;
setting the initial value of the number of incubation patients to a non-zero value based on an epidemic start date and a simulation start date;
setting the initial values of the number of moving-in/moving-out normal population, the number of moving-in/moving-out infected population, the number of disease patients, the number of quarantined patients, the number of confirmed patients, the number of dead patients and the number of cured patients to zero.

48. The simulation method of claim 46, wherein the controlling step comprises at least one of:

for the moving-in behavior, setting a rule involved in the moving-in behavior as:

the number of new moving-in normal population = F1 (a total number of moving-in population; a proportion of disease patients),
the number of new moving-in infected population = F2 (a total number of moving-in population; a proportion of disease patients),
wherein F1() and F2() are rule functions of the moving-in behavior, the total number of moving-in population is data involved in the rule functions of the moving-in behavior, and the proportion of disease patients is a parameter involved in the rule functions of the moving-in behavior;

for the moving-out behavior, setting a rule involved in the moving-out behavior as:

the number of new moving-out normal population = F3 (the number of normal population, the number of incubation patients, the number of disease patients, a total number of moving-out population; a proportion of disease patients),
the number of new moving-out infected population = F4 (the number of normal population, the number of incubation patients, the number of disease patients, the total number of moving-out population; a proportion of disease patients),
wherein F3() and F4() are rule functions of the moving-out behavior, the number of normal population, the number of incubation patients and the number of disease patients are states involved in the rule functions of the moving-out behavior, and the total number of moving-out population is data involved in the rule functions of the moving-out behavior, the proportion of disease patients is a parameter involved in the rule functions of the moving-out behavior;

for the internal infection behavior, setting a rule involved in internal infection behavior as:

the number of new incubation patients = F5 (the number of normal population, the number of disease patients, the number of incubation patients, the internal population flow data; the disease infectivity capability value, the internal infectivity correction coefficient for the predetermined area), wherein F5() is a rule function of the internal infection behavior, the number of normal population, the number of disease patients and the number of incubation patients are states involved in the rule function of the internal infection behavior, and the internal population flow data is data involved in the rule function of the internal infection behavior, the disease infectivity capability value and the internal infectivity correction coefficient for the predetermined area are parameters involved in the rule function of the internal infection behavior;

for the migration infection behavior, setting a rule involved in the migration infection behavior as:

the number of new incubation patients = F6 (the number of moving-in normal population, the number of moving-in infected population; the disease infectivity capability value, the migration infectivity correction coefficient for the predetermined area), wherein F6() is a rule function of the migration infection behavior, the number of moving-in normal population and the number of moving-in infected population are states involved in the rule function of the migration infection behavior, the disease infectivity capability value and the migration infectivity correction coefficient for the predetermined area are parameters involved in the rule function of the migration infection behavior;

for the disease behavior, setting a rule involved in the disease behavior as:

the number of new disease patients = F7 (the number of incubation patients; the incubation period time, a probability distribution of disease patients), wherein F7() is a rule function of the disease behavior, the number of incubation patients is a state involved in the rule function of the disease behavior, and the incubation period time and the probability distribution of the disease patients are parameters involved in the rule function of the disease behavior;

for the quarantined behavior, setting a rule involved in the quarantined behavior as:

the number of new quarantined patients = F8 (the number of new disease patients; the medical resource), wherein F8() is a rule function of the quarantined behavior, the number of new disease patients is a state involved in the rule function of the quarantined behavior, and the medical resource is a parameter involved in the rule function of the quarantined behavior;

for the confirmed behavior, setting a rule involved in the confirmed behavior as:

the number of new confirmed patients = F9 (the number of quarantined patients; the disease characteristic, the medical resource), wherein F9() is a rule function of the confirmed behavior, the number of quarantined patients is a state involved in the rule function of the confirmed behavior, and the disease characteristics and the medical resource are parameters involved in the rule function of the confirmed behavior;

for the dead behavior, setting a rule involved in the dead behavior as:

the number of new dead patients = F10 (the number of confirmed patients; the disease characteristic, the medical resource), wherein F10() is a rule function of the dead behavior, the number of confirmed patients is a state involved in the rule function of the dead behavior, and the disease characteristic and the medical resource are parameters involved in the rule function of the dead behavior;

for the cured behavior, setting a rule involved in the cured behavior as:

the number of new cured patients = F11 (the number of confirmed patients; the disease characteristics, the medical resource), wherein F11() is a rule function of the cured behavior, the number of confirmed patients is a state involved in the rule function of the cured behavior, and the disease characteristic and the medical resource are parameters involved in the rule function of the cured behavior.

49. The simulation method of claim 48, wherein the controlling step comprises setting a rule involved in the behavior that drives the change in the state as:

the number of normal population = the number of normal population + the number of new normal population = the number of normal population + the number of new moving-in normal population - the number of new moving-out normal population + the number of new cured patients;
the number of moving-in normal population = the number of new moving-in normal population;
the number of moving-out normal population = the number of new moving-out normal population;
the number of moving-in infected population = the number of new moving-in infected population;
the number of moving-out infected population = the number of new moving-out population;
the number of incubation patients = the number of incubation patients + the number of new incubation patients - the number of new disease patients + the number of moving-in infected population - the number of moving-out infected population, wherein the number of new incubation patients = the number of incubation patients for the internal infection behavior + the number of incubation patients for the migration infection behavior;
the number of disease patients = the number of disease patients + the number of new disease patients - the number of new quarantined patients;
the number of quarantined patients = the number of quarantined patients + the number of new quarantined patients - the number of new confirmed patients;
the number of confirmed patients = the number of confirmed patients + the number of new confirmed patients - the number of new dead patients - the number of new cured patients;
the number of dead patients = the number of dead patients + the number of new dead patients;
the number of cured patients = the number of cured patients + the number of new cured patients.

50. The simulation method of claim 49, wherein the receiving step, the controlling step and the running step are performed in units of days;

wherein the simulation result data is the number of simulated confirmed patients in the predetermined area output every day, and the reference data is the number of real confirmed patients in the predetermined area every day;
the optimizing step comprises calculating a mean square error between the number of simulated confirmed patients and the number of real confirmed patients for a predetermined number of days, and optimizing the learnable parameters by using an evolutionary algorithm based on the calculated mean square error.

51. A simulation system, comprising:

a user interface module configured to receive an external input;
a control module configured to control a behavior described in a preset simulation logic program based on the external input, wherein the preset simulation logic program describes a state in a simulated item and a behavior that drives a change in the state in code, wherein when the external input comprises a learnable parameter, an optimized value of the learnable parameter is obtained, and the step of controlling the behavior described in the preset simulation logic program based on the external input is performed by using the optimized value as a value of the learnable parameter;
a simulation module configured to run the preset simulation logic program in response to the controlling;
an optimization module configured to optimize the value of the learnable parameter based on output simulation result data.

52. A computer readable storage medium storing instructions that, when executed by at least one computing device, cause the at least one computing device to perform a simulation method according to any one of claims 26 to 50.

100

101                              102

| user interface<br>module |     | control module |

103                              104

| simulation module |     | optimization<br>module |

FIG. 1

data    domain knowledge    event    learnable parameter

user interface module 101

data    domain knowledge    event    learnable parameter

control module 102

rule, parameter, data, state change

simulation module 103

simulation logic program

behavior

state

optimize

optimization module 104

optimization target

output simulation result

reference data

FIG. 2

current state

behavior

rule    +    parameter    +    data

next state

control module 102

rule controller    parameter controller    data controller    state controller

learnable parameter

domain knowledge    domain knowledge    data    event

FIG. 3

| user interface module 101 | | | | | |
|---|---|---|---|---|---|
| start simulation | simulation intervention | pause simulation | switch mode | data import | result display |

| simulation module 103 | control module 102 | optimization module 104 | data management interface module 106 |
|---|---|---|---|
| simulation logic program | rule<br>parameter<br>data<br>state | learnable parameter<br>optimization target<br>optimization algorithm | |

| data management module 105 | | |
|---|---|---|
| preset data | real-time data | user data |

| compute and store resourses 107 |
|---|

FIG. 4

```
┌─────────────────────────┐
│      receiving an       │────────────  501
│    external input       │
└─────────────────────────┘
             │
             ▼
┌─────────────────────────────────────┐
│ controlling a behavior described in │
│ a preset simulation logic program   │────────────  502
│ based on the external input         │
└─────────────────────────────────────┘
             │
             ▼
┌─────────────────────────────────────┐
│  in response to the controlling,    │
│  running the preset simulation      │────────────  503
│  logic program                      │
└─────────────────────────────────────┘
             │
             ▼
┌─────────────────────────────────────┐
│ optimizing a value of a learnable   │
│ parameter based on output simulation│────────────  504
│ result data                         │
└─────────────────────────────────────┘
```

FIG. 5

FIG. 6

# EP 4 141 584 A1

## INTERNATIONAL SEARCH REPORT

International application No.

**PCT/CN2021/084557**

### A. CLASSIFICATION OF SUBJECT MATTER

G05B 17/02(2006.01)i

According to International Patent Classification (IPC) or to both national classification and IPC

### B. FIELDS SEARCHED

Minimum documentation searched (classification system followed by classification symbols)

G05B

Documentation searched other than minimum documentation to the extent that such documents are included in the fields searched

Electronic data base consulted during the international search (name of data base and, where practicable, search terms used)

WPI, EPODOC, CNPAT, CNKI, IEEE: 仿真, 外部输入, 状态, 优化, 学习, 调整, 修改, 编辑, 汽车, 疫情, 传染病, 疾病, 速度, simulat+, external input, condition, state, optimiz+, learn+, study+, adjust+, modify+, revis+, amend+, edit+, vehicle, car, automobile, epidemic, infection, disease, speed

### C. DOCUMENTS CONSIDERED TO BE RELEVANT

| Category* | Citation of document, with indication, where appropriate, of the relevant passages | Relevant to claim No. |
|---|---|---|
| PX | CN 111522255 A (4PARADIGM (BEIJING) TECHNOLOGY CO., LTD.) 11 August 2020 (2020-08-11) description paragraphs 0049-0192 | 1-52 |
| PX | CN 111540478 A (4PARADIGM (BEIJING) TECHNOLOGY CO., LTD.) 14 August 2020 (2020-08-14) description paragraphs 0082-0225 | 1-52 |
| X | CN 110837697 A (SOUTH CHINA UNIVERSITY OF TECHNOLOGY) 25 February 2020 (2020-02-25) description paragraphs 0047-0108 | 1-52 |
| A | CN 102637224 A (NORTHWESTERN POLYTECHNICAL UNIVERSITY) 15 August 2012 (2012-08-15) entire document | 1-52 |
| A | CN 110705078 A (HARBIN ENGINEERING UNIVERSITY) 17 January 2020 (2020-01-17) entire document | 1-52 |
| A | US 5960182 A (KABUSHLKL KALSHA TOSHIBA) 28 September 1999 (1999-09-28) entire document | 1-52 |

☑ Further documents are listed in the continuation of Box C.   ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **10 May 2021** | **25 June 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2021/084557** |

**C. DOCUMENTS CONSIDERED TO BE RELEVANT**

| Category* | Citation of document, with indication, where appropriate, of the relevant passages | Relevant to claim No. |
| --- | --- | --- |
| A | US 2004260531 A1 (BEACH SOLUTIONS LIMITED) 23 December 2004 (2004-12-23) entire document | 1-52 |

Form PCT/ISA/210 (second sheet) (January 2015)

INTERNATIONAL SEARCH REPORT
Information on patent family members

International application No.

**PCT/CN2021/084557**

| Patent document cited in search report | | | Publication date (day/month/year) | Patent family member(s) | | | Publication date (day/month/year) |
|---|---|---|---|---|---|---|---|
| CN | 111522255 | A | 11 August 2020 | None | | | |
| CN | 111540478 | A | 14 August 2020 | None | | | |
| CN | 110837697 | A | 25 February 2020 | None | | | |
| CN | 102637224 | A | 15 August 2012 | None | | | |
| CN | 110705078 | A | 17 January 2020 | None | | | |
| US | 5960182 | A | 28 September 1999 | JP | H10187789 | A | 21 July 1998 |
| US | 2004260531 | A1 | 23 December 2004 | WO | 03023658 | A2 | 20 March 2003 |
| | | | | GB | 2383654 | A | 02 July 2003 |
| | | | | AU | 2002331933 | A1 | 24 March 2003 |

Form PCT/ISA/210 (patent family annex) (January 2015)

**EP 4 141 584 A1**

**REFERENCES CITED IN THE DESCRIPTION**

**Patent documents cited in the description**

- CN 202010322898X
- CN 202010322919